# FASCICULE DE BREVET EUROPEEN

(11) **EP 1 536 806 B1**
(45) Date de publication et mention de la délivrance du brevet: **22.08.2007**
(21) Numéro de dépôt: 03761660.4
(22) Date de dépôt: 27.06.2003
(51) Int. Cl.: A61K 31/7048, A23L 1/29, A61P 19/08

(54) **UTILISATION DE L'HESPERIDINE OU L'UN DE SES DERIVES POUR LA FABRICATION D'UN MEDICAMENT DESTINE A STIMULER LA FORMATION OSSEUSE**
VERWENDUNG VON HESPERIDIN ODER EINEM SEINER DERIVATE ZUR HERSTELLUNG EINES MEDIKAMENTS FÜR DIE STIMULIERUNG DER KNOCHENBILDUNG
USE OF HESPERIDINE OR ONE OF ITS DERIVATIVES FOR MAKING A MEDICINE FOR BONE FORMATION STIMULATION

(30) Priorité: 28.06.2002 FR 0208171
(43) Date de publication de la demande: 08.06.2005
(73) Titulaire: INSTITUT NATIONAL DE LA RECHERCHE AGRONOMIQUE (INRA), 75007 Paris Cédex 07 (FR)
(72) Inventeur: HORCAJADA, Marie-Noelle, F-63360 Gerzat (FR); COXAM, Véronique, F-63122 Ceyrat (FR); MORAND, Christine, F-63350 Crevant Laveine (FR); DAVICCO, Marie-Jeanne, F-63400 Chamalières (FR)
(74) Mandataire: Catherine, Alain
(86) Numéro de dépôt international: PCT/FR2003/002005
(87) Numéro de publication internationale: WO 2004/002496

(56) Documents cités:
- EP-A- 0 719 554
- EP-A- 0 938 899
- EP-A- 1 127 572
- WO-A-02/17909
- US-A- 5 506 211
- DATABASE CA [Online] CHEMICAL ABSTRACTS SERVICE, COLUMBUS, OHIO, US; 2001, KISE, MITSUO ET AL: "Vitamin K compositions containing flavonoids as stabilizers" XP002234081 accession no. STN Database accession no. 134:316087 CA & JP 2001 114675 A (FANCHEL K. K., JAPAN) 24 avril 2001 (2001-04-24)

## Description

### DOMAINE DE L'INVENTION

L'invention se rapporte au domaine du maintien ou du rétablissement de l'équilibre du métabolisme osseux chez l'homme ou l'animal, notamment pour la prévention ou le traitement de troubles liés à un déséquilibre du métabolisme osseux grâce à l'apport nutritionnel ou à l'administration thérapeutique d'une composition stimulant la formation de l'os et/ou inhibant la résorption osseuse.

### ETAT DE LA TECHNIQUE.

L'os n'est pas un tissu statique. L'os subit un remodelage constant par destruction et synthèse *de novo* du tissu osseux dans un processus complexe impliquant deux types principaux de cellules, respectivement les ostéoblastes qui produisent du tissu osseux nouveau et les ostéoclastes qui détruisent l'os.

Les activités de ces cellules sont régulées par un grand nombre de cytokines et de facteurs de croissance, dont la plupart ont été identifiés et clonés comme cela est décrit dans la revue générale de Mundy (Mundy, G.R., 1996, Clin. Orthop., vol.324:24-28; Mundy, G.R., 1993, J.Bone Miner Res, vol.8 : S 505 - S 510).

Les ostéoblastes, cellules responsables de la formation de l'os, se différencient à partir de cellules précurseurs et expriment et secrètent de nombreuses enzymes et de nombreuses protéines de structure de la matrice de l'os, y compris le collagène de type 1, l'ostéocalcine, l'ostéopontine et la phosphatase alcaline (Stein G. et al., 1990, Curr. Opin Cell Biol. vol.2: 1018-1027; Harris S et al. , 1994, J. Bone Miner Res Vol.9:855-863).

Les ostéoblastes synthétisent aussi de nombreux peptides régulateurs de croissance, y compris les peptides BMPs (pour "Bone Morphogenetic Proteins »), qui sont stockés dans la matrice osseuse, et qui sont vraisemblablement responsables de la formation normale de l'os.

Comme la phosphatase alcaline, l'ostéocalcine et l'ostéopontine, les peptides BMPs sont exprimés par des ostéoblastes en culture au moment où ces cellules prolifèrent et se différencient.

Les ostéoclastes sont des cellules multinucléées qui sont responsables de la perte osseuse, dans un processus communément désigné la résorption osseuse.

Chez un individu en bonne santé, homme ou animal adulte, l'action conjointe des ostéoblastes et des ostéoclastes permet un maintien dans le temps de la masse osseuse et assure simultanément un remodalage du tissu osseux par résorption et synthèse *de novo* de l'os.

Chez un sujet adulte sain, la vitesse de formation des ostéoclastes et des ostéoblastes est telle qu'il y a un équilibre entre la formation de l'os et la résorption osseuse. Cependant, chez les individus ostéoporotiques, il se produit un déséquilibre dans le processus de remodelage osseux, ce qui aboutit à une perte d'os qui se fait à une vitesse plus rapide que sa vitesse de formation. Bien que ce déséquilibre existe, dans une certaine mesure, chez la plupart des individus à mesure qu'ils vieillissent, il est beaucoup plus sévère et se produit à un âge plus jeune chez les individus ostéoporotiques.

Ainsi, chez l'homme et les autres mammifères, une grande diversité de troubles sont liés à un métabolisme anormal de la résorption osseuse ou de la formation de l'os, entraînant un déséquilibre du métabolisme ou remodelage osseux.

On observe notamment une grande hétérogénéité dans la valeur du pic de masse osseuse selon les individus, cette hétérogénéité étant due à des grandes variations dans le processus de croissance osseuse dès le plus jeune âge. Ainsi, la masse osseuse maximale atteinte au début de l'âge adulte, aussi désignée pic de masse osseuse, est-elle très variable d'un individu à l'autre. Avec l'âge, les individus possédant une valeur faible de pic de masse osseuse, et qui subissent une perte osseuse due au vieillissement, sont désavantagés. Ainsi, quel que soit l'individu, un traitement préventif précoce de la perte osseuse est préconisé, de manière à réduire le plus possible les déséquilibres du remodelage osseux qui se produisent de manière non pathologique avec le vieillissement, notamment les risques de fractures osseuses précoces.

Parmi les désordres pathologiques liés à un déséquilibre du métabolisme osseux, on peut citer notamment les troubles ou les pathologies tels que l'ostéoporose, la maladie de Paget, la perte osseuse ou l'ostéolyse observée à proximité d'une prothèse, les maladies de l'os métastatique, l'hypercalcémie due à un cancer, les myélomes multiples, et les maladies périodontales.

Certains des troubles ou pathologies du métabolisme osseux peuvent être provoqués après une immobilisation de longue durée, par exemple une hospitalisation de longue durée, ou encore après un séjour en apesanteur.

Parmi les troubles liés à une résorption osseuse anormale , le plus commun est l'ostéoporose, dont la manifestation la plus fréquence est observée chez les femmes, après le début de la ménopause. L'ostéoporose est une maladie squelettique systémique caractérisée par une réduction de la masse osseuse et une détérioration de la microarchitecture du tissu osseux, associées à une augmentation de la fragilité de l'os et de sa susceptiblité à la fracture.

Il existe d'autres facteurs susceptibles d'accroître la perte osseuse conduisant à l'ostéoporose, telle que la consommation de cigarettes, l'abus d'alcool, un mode de vie sédentaire, un faible apport en calcium, une alimentation déséquilibrée ou encore une carence en vitamine D.

Du fait que l'ostéoporose, comme d'autres troubles associés à une perte osseuse, constitue un trouble chronique, sa prévention et son traitement doivent être envisagés sur une longue durée.

Il est couramment admis qu'une prise en charge précoce doit être privilégiée puisque les deux phases critiques pour le capital osseux sont :
- la période de croissance permettant l'acquisition de la masse osseuse maximale (pic de masse osseuse) ;
- le vieillissement conditionnant la vitesse de perte de la masse osseuse.

La prévention de l'ostéoporose ne doit donc plus être restreinte à l'individu âgé.

Par ailleurs, chez l'homme ou l'animal, il existe de nombreux états caractérisés par la nécessité d'accroître la formation osseuse. Par exemple, dans le cas de fractures osseuses, il est nécessaire de stimuler la croissance osseuse afin d'accélérer une réparation complète de l'os. Ce besoin est également présent dans les maladies périodontales, les maladies métastatiques de l'os, les maladies ostéolytiques et les états dans lesquels on recherche une réparation du tissu conjonctif, par exemple pour la cicatrisation ou la régénération des défauts ou des traumatismes du cartilage. On recherche également la stimulation d'une croissance osseuse dans le cas d'hyperparathyroïdisme primaire et secondaire, ainsi que dans l'ostéoporose associée au diabète et dans l'ostéoporose associée aux glucocorticoïdes.

Il existe aujourd'hui de nombreux composés actifs sur la stimulation de la formation de l'os ou l'inhibition de la résorption osseuse, parmi lesquels on peut citer la famille des composés polyphosphonates (brevet européen n°EP 210 728), les oxazolidinones thioamide (demande de brevet US publiée sous le n°2002/0010341), les composés amino bis-phosphonates (demande de brevet US publiée sous le n°2001/0046977), ou encore les composés isoflavones (demande de brevet US publiée sous le n°2002/0035074). On a également proposé l'utilisation d'extrait d'une plante du genre *Lycopersicon* (demande de brevet US publiée sous le n°2002/0009510).

JP 2001114675 décrit l'utilisation de la vitamine K2 en combinaison avec d'autres composants pour le traitement de l'ostéoporose.

EP 1127572 concerne l'utilisation de flavones pour le traitement de maladies liées à la cyclooxigenase-2 (COX) et au NFₖB.

Bien qu'il existe aujourd'hui une grande diversité de composés actifs pour stimuler la formation osseuse et/ou inhiber la résorption osseuse, il existe un besoin constant pour de nouveaux composés actifs, notamment du fait du succès limité des traitements actuels.

De plus, compte-tenu du caractère chronique de certains états provoqués par un déséquilibre du métabolisme osseux, il existe un besoin pour de nouveaux composés actifs susceptibles de pouvoir être utilisés pendant une longue durée chez l'homme ou l'animal, et susceptibles de se présenter sous la forme d'un apport nutritionnel, par exemple sous la forme d'une composition nutritionnelle.

### SOMMAIRE DE L'INVENTION.

De manière surprenante, le demandeur a montré que l'hespéridine, qui est un composé de la famille des flavonoïdes, est capable d'agir sur le métabolisme osseux en stimulant la formation de l'os et en inhibant la résorption osseuse.

Il est donc fourni selon l'invention, une composition nutritionnelle et une composition pharmaceutique à usage humain ou vétérinaire comprenant, à titre de composé actif, le composé hespéridine ou l'un de ses dérivés.

L'invention est également relative à l'utilisation du composé hespéridine ou de l'un de ses dérivés pour la fabrication d'une composition destinée à stimuler la formation de l'os et/ou à inhiber la résorption osseuse chez l'homme ou l'animal, en particulier le chien, le chat ou le cheval.

Selon un premier aspect, l'utilisation ci-dessus est caractérisée en ce que ladite composition est une composition nutritionnelle adaptée pour une administration orale.

Selon un second aspect, l'utilisation ci-dessus est caractérisée en ce que ladite composition est une composition pharmaceutique pour une administration orale, parentérale ou intra-veineuse.

### DESCRIPTION DES FIGURES.

La **figure 1** illustre le maintien d'une composition corporelle (% de masse grasse par rapport au poids corporel) équivalente entre les différents lots d'animaux, respectivement des animaux normaux (TSH), des animaux ovariectomisés (TOVX), des animaux normaux traités par l'hespéridine (HpSH) et des animaux ovariectomisés traités par l'hespéridine (HpOVX). En ordonnées, le pourcentage de masse grasse.

La **figure 2** illustre l'augmentation de la densité fémorale métaphysaire chez les rats intacts ou ovariectomisés recevant le régime hespéridine, par comparaison à ceux soumis à une alimentation standard. En ordonnées, la densité osseuse, en g/cm³.

La **figure 3** illustre la prévention, par l'hespéridine, de l'augmentation de la résorption osseuse induite par l'ovariectomie, évaluée par la mesure de l'excrétion urinaire de désoxypyridinoline. En ordonnées, mesure du rapport molaire désoxypyridinole/créatinine dans l'urine.

La **figure 4** illustre la stimulation de l'activité ostéoblastique (évaluée par la mesure des taux plasmatiques d'ostéocalcine) chez les rates traitées par rapport aux animaux témoins. En ordonnées, taux plasmatique d'ostéocalcine, exprimé en mg/l,

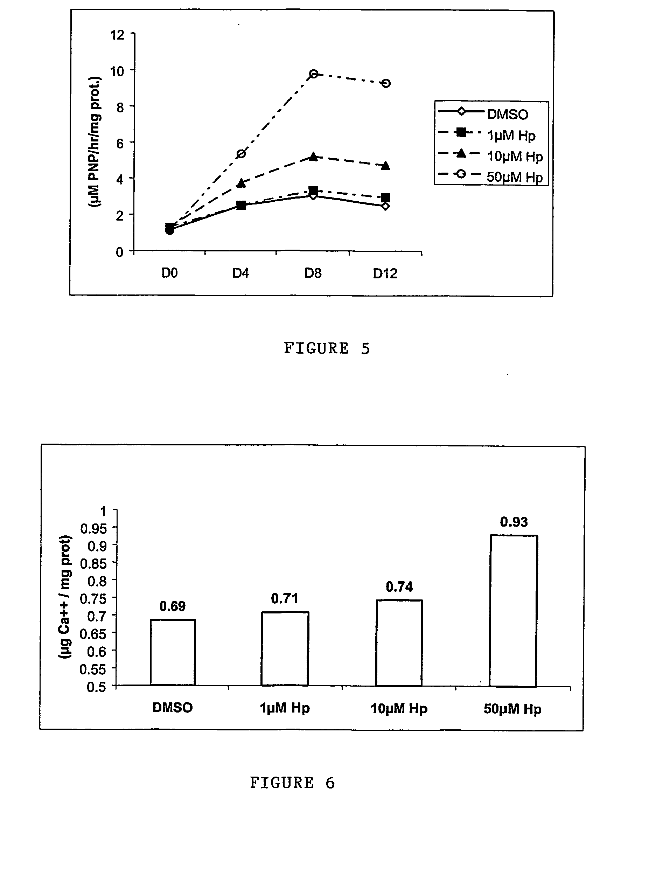

La **figure 5** illustre la cinétique de la différentiation ostéoblastique (augmentation de l'activité de la phosphatase alcaline) stimulée par l'hespérétine à 10 et 50µM sur les cellules hPOB-tert à passage 31. En ordonnées, l'activité de la phosphatase alcaline est exprimée en µM de paranitrophénol/heure/mg de protéines. En abscisses, le temps est exprimé en jours.

La **figure 6** illustre la stimulation de l'accumulation calcique dans les cellules hPOB-tert (à passage 31) par l'hespéritine (à 50µM), après 21 jours d'incubation. En ordonnées, mesure du rapport calcium Ca⁼⁼/protéines, exprimée en µgCa⁺⁺/mg protéines.

La **Figure 7** illustre le contrôle de la consommation alimentaire au cours du temps chez des rates ovariectomisées non traitées (OVX Témoin) ou traitées avec l'hespéridine (OVX Hesp), et chez des rates normales témoin (SH Témoin) ou traitées avec l'hespéridine (SH Hesp). En abscisse : temps, de deux jours en deux jours ; En ordonnées : grammes d'aliments consommés.

La **Figure 8** illustre le profil de l'évolution du poids des rates dans les lots (SH), (SH Hesp), (OVX) et (OVX Hesp). En abscisse : Nombre de jours ; en ordonnées : poids moyen des animaux +/- déviation standard.

La **Figure 9** illustre la composition corporelle, en pourcentage de masse grasse, des rates dans les lots (SH), (SH Hesp), (OVX) et (OVX Hesp). En abscisse : lot d'animaux; en ordonnées : composition corporelle, exprimée en pourcentage de masse grasse.

La **Figure 10** illustre le poids de l'utérus des rates dans les lots (SH), (SH Hesp), (OVX) et (OVX Hesp). En abscisse : lot d'animaux ; en ordonnées: poids moyen des utérus, exprimé en gramme.

La **Figure 11** illustre la densité fémorale totale (T-BMD) chez les rates dans les lots (SH), (SH Hesp), (OVX) et (OVX Hesp). En abscisse : lot d'animaux ; en ordonnées : densité minérale fémorale, exprimée en g/cm².

La **Figure 12** illustre la densité minérale de la métaphyse proximale fémorale (M-BMDp) chez les rates dans les lots (SH), (SH Hesp), (OVX) et (OVX Hesp). En abscisse : lot d'animaux ; en ordonnées : densité minérale de la métaphyse proximale fémorale, exprimée en g/cm².

La **Figure 13** illustre la densité minérale diaphysaire fémorale (D-BMD) chez les rates dans les lots (SH), (SH Hesp), (OVX) et (OVX Hesp). En abscisse : lot d'animaux ; en ordonnées : densité minérale diaphysaire fémorale, exprimée en g/cm².

La **Figure 14** illustre la charge à la rupture fémorale chez les rates dans les lots (SH), (SH Hesp), (OVX) et (OVX Hesp). En abscisse : lot d'animaux ; en ordonnées : charge à la rupture fémorale, exprimée en Newtons (N).

### DESCRIPTION DETAILLEE DE L'INVENTION.

Il est fourni selon l'invention, une composition nutritionnelle et une composition pharmaceutique à usage humain ou vétérinaire destinées à stimuler la formation de l'os et/ou à inhiber la résorption osseuse chez l'homme ou l'animal et comprenant, à titre de composé actif, le composé hespéridine ou l'un de ses dérivés.

L'hespéridine est un composé glucoside naturel retrouvé principalement dans les agrumes, c'est-à-dire les fruits du genre *Citrus.* L'hespéridine est présente majoritairement dans la peau des agrumes mais est également retrouvée en grandes quantités dans la pulpe et donc le jus des agrumes, y compris les oranges et les citrons.

L'hespéridine est un composé glucosylé comprenant un noyau flavanone d'hespéritine (3',5',5-trihydroxy-4'-méthoxyflavanone) auquel est liée de manière covalente une partie glucosidique de rutinose L-rhamnosyl-(α 1→6)-glucose) fixée sur le groupe hydroxyle présent sur le carbone en position 7 de l'hespéritine.

Le composé hespéridine est un composé de la famille des flavonoïdes qui est utilisé couramment en association avec la vitamine C, afin de potentialiser l'activité biologique de la vitamine C, notamment en réduisant la dénaturation de la vitamine C par oxydation. Dans cette application, l'hespéridine est proposée comme agent auxiliaire dans le traitement de pathologies cardio-vasculaires, notamment de l'hypertension et des hémorragies.

L'hespéridine a également été décrite comme agent chondro-protecteur, du fait de sa capacité à inhiber la destruction de la matrice des chondrocytes dérivés du cartilage (demande de brevet n°EP 633 022).

L'hespéridine a également été proposée dans l'état de la technique comme agent pour prévenir la pigmentation de la peau (demande de brevet allemand n°DE 19742 025), comme agent inhibiteur de la HMG-CoA réductase (brevet US N°5,763,414) ou de l'acyl CoA-cholestérol-O-acyl' transférase (demande PCT N°WO 99/21.549) ou encore de l'agrégation plaquettaire (demande de brevet coréen n°KR 276 979).

De manière surprenante, il a été montré selon l'invention que l'hespéridine stimule la formation de l'os et inhibe la résorption osseuse.

Comme cela est illustré par les exemples, l'hespéridine induit une augmentation de la densité du tissu osseux métaphysaire (os trabéculaire) et de la densité de l'os diaphysaire (os cortical), induisant ainsi une amélioration de la minéralisation osseuse.

L'hespéridine stimule également l'accrétion osseuse comme en témoigne la forte augmentation des taux plasmatiques d'ostéocalcine observés chez les animaux ayant reçu une alimentation supplémentée en ce composé.

Il a également été montré que l'hespéridine provoque un arrêt de multiplication des cellules précurseurs des ostéoblastes ainsi que leur différenciation en ostéoblastes matures, comme cela est visualisé par l'induction d'une augmentation de l'activité de la phosphatase alcaline. De même, l'hespéridine induit une augmentation de l'accumulation du calcium dans les ostéoblastes.

De plus, l'hespéridine inhibe la résorption osseuse chez des rats ovariectomisés, qui est le modèle expérimental animal de référence mimant l'ostéoporose humaine.

En particulier, il est montré selon l'invention que l'hespéridine permet le maintien du capital osseux au cours du vieillissement chez les individus ayant terminé leur croissance, notamment chez les individus en situation de carence hormonale, comme cela est observé chez les rates ovariectomisées âgées de 6 mois, qui sont un modèle mimant la situation physiologique de ménopause..

Ces deux activités conjointes de l'hespéridine, respectivement de formation de l'os et d'inhibition de la résorption osseuse, lui confèrent une grande utilité en tant que composé actif chez l'homme ou l'animal pour maintenir ou rétablir l'équilibre du métabolisme osseux, c'est-à-dire :
- soit maintenir constante au cours du temps, avec l'âge, les activités des cellules ostéoblastiques et ostéoclastiques et ainsi prévenir des troubles du métabolisme osseux ;
- soit remédier à un déséquilibre du métabolisme osseux, par exemple dans des troubles comme l'ostéoporose, ou encore stimuler la régénération osseuse, par exemple en cas de fêlure ou fracture de l'os.

La présente invention a donc pour objet l'utilisation du composé hespéridine ou de l'un de ses dérivés pour la fabrication d'une composition apte à maintenir ou à rétablir l'équilibre du métabolisme osseux chez l'homme ou l'animal, en stimulant la formation de l'os et/ou en inhibant la résorption osseuse.

Par hespéridine, on entend le composé (S)-7-[[6-0-(6-deoxy-α-L-mannopyranosyl)-β-D-glucopyranosyl]oxy]-2,3-dihydro-5-hydroxy-2-(3-hydroxy-4-methoxyphenyl)-4H-1-benzopyran-4-one.

Par « dérivé » de l'hespéridine, on entend selon l'invention les composés suivants:
- le composé hespérétine, constitué du noyau flavanone non glycosylé de l'hespéridine, qui a la formule suivante : (S)-2,3-dihydro-5,7-dihydroxy-2-(3-hydroxy-4-méthoxyphényl)-4H-1-benzopyran-4-one ;3',5,7-trihydroxy -4'-méthoxyflavanone ;
- l'α-glucosyl-hespéridine, qui comporte une chaîne de 1 à 20 résidus de glucose liés entre eux par une liaison 1,4, la chaîne de résidus glucose étant liée elle-même par une liaison de type 1,4 en position 4 du résidu glucose de l'hespéridine ; ces dérivés de l'hespéridine et leur procédé de préparation sont décrits notamment dans la demande de brevet N°EP 0 825 196 et dans le brevet US N°6,048,712 ;
- les composés méthyl-hespéridine, notamment le composé 3¹-méthyl-7-(rhamnosyl-2-méthyl-glucosyl)hespéridine et le composé 3¹-méthylhespéridine, ces composés, ainsi que leur procédé de préparation étant décrits dans le brevet US N°858,784) ;
- les conjugués d'hespérétine et de sulfate ou de glucuronide, qui sont retrouvés, avec l'hespérétine, comme produits de la métabolisation de l'hespéridine dans la circulation sanguine.

Par « stimulation de la formation de l'os », on entend, selon l'invention, la capacité de l'hespéridine ou de l'un de ses dérivés à stimuler l'activité des ostéoblastes et favoriser ainsi la synthèse de la trame protéique de l'os et le dépôt des minéraux, surtout du calcium, sur cette trame protéique, c'est-à-dire à stimuler la minéralisation de l'os, encore appelée accrétion osseuse.

Pour vérifier que l'apport en hespéridine a un homme ou un animal, en particulier un mammifère, stimule la formation de l'os, l'homme du métier pourra notamment avoir recours à des mesures conventionnelles de densitométrie et vérifier que l'apport d'hespéridine ou de l'un de ses dérivés, à une dose donnée, induit un accroissement de la densité osseuse.

L'homme du métier peut également avoir recours à l'un quelconque des autres tests également décrits dans les exemples, telles que la mesure résistance de l'os à la rupture ou encore à la mesure de l'activité de la phosphatase alcaline et la mesure de l'accumulation du calcium dans des cellules ostéoblastes.

Par « inhibition de la résorption osseuse », on entend selon l'invention, une inhibition de l'activité de destruction du tissu osseux par les cellules ostéoclastes. Pour vérifier que l'apport d'hespéridine ou de l'un de ses dérivés, chez l'homme ou l'animal, inhibe la résorption osseuse, l'homme du métier peut mesurer l'excrétion urinaire de désoxypyridinoline, comme décrit dans les exemples, une diminution de l'expression de la désoxypyridinoline étant le reflet d'une inhibition de la résorption osseuse.

L'apport d'hespéridine ou de l'un de ses dérivés à un organisme animal induit simultanément une stimulation de la formation de l'os et une inhibition de la résorption osseuse, l'accroissement global de la minéralisation osseuse, et donc de la densité de l'os, étant le résultat de l'induction de ces deux mécanismes.

Pour déterminer si un sujet présente un état de masse osseuse réduite, et nécessite en conséquence un apport d'hespéridine ou de l'un de ses dérivés, l'homme du métier pourra se référer notamment au rapport de l'Organisation Mondiale de la Santé (OMS) de 1994 intitulé « Assessment of fracture risk and its application to screening for post-menopausal osteoporosis « WHO Technical Series-843).

Une composition nutritionnelle ou une composition thérapeutique contenant de l'hespéridine ou l'un de ses dérivés comme composé actif est destinée tout d'abord à la prévention de la perte osseuse due à un déséquilibre dans le remodelage du tissu osseux, chez l'homme ou l'animal, en particulier chez un mammifère non humain, notamment un mammifère domestique comme le chien ou le chat, ou encore un cheval, en particulier un pur-sang.

Une composition telle que définie ci-dessus est également destinée à favoriser la croissance osseuse chez les individus jeunes afin d'obtenir des individus possédant une grande densité osseuse et, si possible, concomitamment un pic de masse osseuse élevé. En particulier, une composition selon l'invention est utile pendant la phase de croissance de l'homme ainsi que des autres mammifères, notamment des chiens de grandes races ou à pedigree, ou encore les chevaux de course.

La composition contenant de l'hespéridine ou l'un de ses dérivés est aussi destinée aux individus présentant des symptômes de déficit osseux, ou susceptibles de souffrir de déficit osseux, c'est-à-dire d'un déséquilibre du rapport entre la formation de l'os et la résorption osseuse lequel, s'il se poursuit, induit une diminution de la masse osseuse. Une composition selon l'invention est également destinée aux individus présentant des symptômes de déficit osseux résultant d'une fracture, d'une intervention chirurgicale ou encore d'une maladie dentaire.

En particulier, une composition nutritionnelle ou pharmaceutique à destination humaine ou vétérinaire selon l'invention est utile dans le traitement de pathologies telles que l'ostéoporose, la maladie de Paget, la perte osseuse ou l'ostéolyse observée à proximité d'une prothèse, les maladies de l'os métastatique, l'hypercalcémie due à un cancer, les myélomes multiples, les maladies periodontales ou encore l'ostéoarthrite.

La composition selon l'invention peut se présenter sous la forme d'une composition nutritionnelle ou encore sous la forme d'une composition pharmaceutique, comme cela est décrit ci-après.

Il a également été montré selon l'invention que l'hespéridine induit l'arrêt de la multiplication et provoque la différenciation des cellules ostéoblastes sans nécessiter la présence d'agents auxiliaires tels que des facteurs de différenciation ostéoblastiques comme la vitamine D3 et la déxaméthasone, au contraire de nombreux composés anti-ostéoporose décrits dans l'état de la technique. Cette observation expérimentale souligne l'intérêt de l'hespéridine et de ses dérivés pour stimuler la formation de l'os et inhiber la résorption osseuse, puisque ces composés sont biologiquement actifs utilisés seuls et ne nécessitent pas en conséquence d'être apportés à l'organisme sous la forme d'une association de l'hespéridine ou de l'un de ses dérivés avec un autre composé telle qu'une vitamine.

De plus, le fait que l'hespéridine induise une augmentation non seulement de la densité de l'os trabéculaire, mais également de la densité de l'os cortical démontre le haut niveau d'activité biologique de cette molécule. En effet, l'os trabéculaire, qui forme la tête de l'os et qui est fortement vascularisé, est le lieu privilégié des échanges de calcium entre l'os et la circulation sanguine, alors que l'os cortical, qui forme le corps droit de l'os et qui est très peu vascularisé, est beaucoup moins susceptible d'être affecté rapidement de changement dans sa minéralisation.

### Compositions nutritionnelles contenant de l'hesperidine ou l'un de ses dérives.

Comme cela a déjà été mentionné ci-dessus, de nombreux troubles liés à un déséquilibre du métabolisme osseux, telle que l'ostéoporose, évoluent progressivement sur une longue période de temps et nécessitent des traitements chroniques. Leur prévention ou leur traitement peut donc être réalisé grâce à un apport régulier d'hespéridine ou de l'un de ses dérivés, préférentiellement sous la forme d'une composition nutritionnelle.

De même, un apport nutritionnel régulier d'hespéridine à des individus jeunes en croissance, hommes ou animaux, est de nature à permettre l'obtention d'une grande densité osseuse et un pic de masse osseuse élevé, par stimulation de la formation de l'os, lorsque ces individus atteignent l'âge adulte.

Egalement, un apport nutritionnel régulier d'hespéridine est utile pour prévenir la perte osseuse qui se produit avec le vieillissement.

L'invention a aussi pour objet une composition nutritionnelle pour stimuler la formation de l'os et/ou inhiber la résorption osseuse, caractérisée en ce qu'elle comprend, à titre de composé nutritionnel actif, le composé hespéridine ou l'un de ses dérivés.

Par « composition nutritionnelle », on entend selon l'invention, une composition contenant de l'hespéridine ou l'un de ses dérivés et constituant une composition alimentaire ou encore un supplément alimentaire ne possédant pas les caractéristiques d'un médicament.

Les différentes utilisations du composé hespéridine ou de l'un de ses dérivés pour la fabrication d'une composition nutritionnelle seront définies ci-après en relation avec les caractéristiques techniques de ladite composition nutritionnelle.

Une composition nutritionnelle selon l'invention est préférentiellement adaptée pour une administration orale.

Selon un premier aspect, une composition nutritionnelle selon l'invention est un aliment diététique utilisé pour le maintien d'une bonne santé de l'homme ou l'animal qui l'ingère. Une telle composition nutritionnelle est également communément désignée « aliment fonctionnel », lequel est destiné à être consommé, soit comme partie intégrante du régime alimentaire, soit comme supplément alimentaire, mais dont le contenu en hespéridine ou en l'un de ses dérivés implique un rôle physiologique allant au-delà de la fourniture des besoins nutritifs de base.

Selon un premier aspect, ladite composition nutritionnelle est destinée à stimuler la formation de l'os chez les individus jeunes en phase de croissance.

Selon un second aspect, ladite composition nutritionnelle est destinée à prévenir la perte osseuse qui se produit avec le vieillissement.

Selon un troisième aspect, que ladite composition nutritionnelle est destinée à prévenir ou traiter des troubles liés à un déséquilibre du rapport entre la formation de l'os et la résorption osseuse.

Selon un quatrième aspect, ladite composition nutritionnelle est destinée à traiter un déficit osseux résultant d'une fracture.

Selon encore un autre aspect, que ladite composition nutritionnelle est destinée à prévenir ou à traiter des pathologies choisies parmi l'ostéoporose, la maladie de Paget, la perte osseuse ou l'ostéolyse observée à proximité d'une prothèse, les maladies de l'os métastatique, l'hypercalcémie due à un cancer, les myélomes multiples, les maladies periodontales ou l'ostéoarthrite.

Une composition nutritionnelle selon l'invention, caractérisée en ce qu'elle comprend le composé hespéridine ou l'un de ses dérivés, en tant que composé actif, peut se présenter sous une grande diversité de formes de compositions alimentaires et de boissons, y compris des jus, préférentiellement des jus de fruits, des yaourts, des glaces, des fromages, des produits cuits tels que le pain, les biscuits et les gâteaux, des produits laitiers, des desserts, des produits de confiserie, des barres de céréales, des céréales pour le petit déjeuner, des produits d'assaisonnement des aliments (notamment épices et sauces), salades de fruits, compotes de fruits, etc..

Une composition nutritionnelle selon l'invention, caractérisée en ce qu'elle comprend le composé hespéridine ou l'un de ses dérivés, en tant que composé actif, peut aussi se présenter sous la forme d'une grande diversité de produits destinés à l'alimentation animale, en particulier pour le chien ou le chat, qu'ils soient sous forme humide, sous forme semi-humide ou sous forme sèche , notamment sous la forme de croquettes.

Comme cela a été décrit précédemment, l'hespéridine est un composé retrouvé naturellement dans les agrumes du genre *Citrus,* en particulier dans les oranges et les citrons, ce composé étant contenu majoritairement dans la peau des fruits, mais également retrouvé en quantités significatives dans la pulpe, comme cela est décrit notamment par MANSELL et al. (MANSELL R L et al., 1983, J. Agric. Food Chem., vol.31:156-162), ROUSEFF et al. (ROUSEFF R L et al., 1987, J. Agric. Food Chem, vol.35:1027-1030) ou encore GIL-IZQUIERDO et al. (GIL-IZQUIERDO A et al., 2001, J. Agric. Food Chem. , vol.49: 1035-1041).

Selon un premier aspect, une composition nutritionnelle selon l'invention peut se présenter sous la forme d'un jus d'agrume, en particulier un jus d'orange ou un jus de citron, le cas échéant sous la forme d'un concentré de jus d'agrume.

Selon un second aspect, une composition nutritionnelle selon l'invention peut consister en une composition alimentaire enrichie en agrume ou en extrait d'agrume, en particulier d'orange ou de citron.

Selon un troisième aspect, une composition nutritionnelle selon l'invention se présente sous la forme de tout produit, en particulier toute boisson, aromatisée avec des zestes d'agrume, du jus d'agrume, de la pulpe d'agrume ou tout autre extrait d'agrume, y compris l'orange et le citron.

Ainsi, une composition nutritionnelle selon l'invention, qui se présente sous une forme liquide ou sous une forme solide, notamment sous la forme d'une poudre, peut consister en un produit d'extraction obtenu à partir de la peau ou de la pulpe d'un agrume, ou encore comprendre un produit d'extraction obtenu à partir de la peau ou de la pulpe d'un agrume.

Notamment, une composition nutritionnelle selon l'invention se présente sous la forme d'une boisson, par exemple de l'eau minérale, à laquelle a été ajoutée un extrait de la peau ou de la pulpe d'un agrume, y compris d'une orange ou d'un citron.

Selon encore un autre aspect, le produit d'extraction peut être initialement enrichi en hespéridine si l'on met en oeuvre un procédé d'extraction spécialement adapté pour atteindre un tel objectif, tel que le procédé décrit dans la demande de brevet japonais N°JP 8 188 593, dans lequel le produit de pressage des fruits du genre *Citrus* est ajusté à pH 11,5 - 12,5 puis le liquide obtenu après pressage et ajustement du pH est centrifugé, avant un second ajustement du pH à pH 5-5,5 puis un chauffage et une dernière étape de centrifugation préalable à la récupération du produit enrichi en hispéridine. Un autre procédé d'extraction susceptible d'être utilisé pour l'obtention d'un extrait enrichi en hespéridine est celui décrit dans la demande de brevet britannique n°GB 486.898.

Pour l'obtention d'un produit d'extraction enrichi en hespéridine, à partir d'agrumes, et plus spécifiquement à partir d'orange ou de citron, l'homme du métier pourra aussi avantageusement se référer aux procédés décrits respectivement dans le brevet US N°2,400,693 et US 2,442,110.

Selon un autre aspect, dans une composition nutritionnelle selon l'invention, l'hesperidine ou l'un de ses dérivés produit par synthèse chimique.

De préférence, une composition nutritionnelle selon l'invention comprend une quantité de composé hespéridine ou de l'un de ses dérivés adaptée à une administration orale quotidienne comprise entre 0,01 mg et 500 mg.

Pour une consommation chez l'homme, une composition nutritionnelle selon l'invention comprend une quantité de composé actif adaptée à un apport quotidien en hespéridine ou en l'un de ses dérivés, fourni par ladite composition, comprise entre 0,01 mg et 500 mg, de préférence entre 0,1 mg et 500 mg et de manière tout à fait préférée entre 1 mg et 500 mg.

Pour une consommation par un animal, spécifiquement un mammifère non humain, y compris le chien, le chat ou le cheval, une composition nutritionnelle selon l'invention est adaptée à une administration quotidienne de composé actif, fournie par ladite composition, comprise entre 1 mg et 500 mg, de préférence 10 mg et 500 mg d'hespéridine ou l'un de ses dérivés.

A titre illustratif, une eau minérale supplémentée en hespéridine ou l'un de ses dérivés contiendra de 0,01 à 500 mg de composés actifs par litre, en estimant la consommation humaine moyenne quotidienne d'eau minérale comme étant d'environ 1 litre.

Selon encore un autre aspect, la composition nutritionnelle ci-dessus peut comprendre d'autres composés nutritionnels, en combinaison avec l'hespéridine ou l'un de ses dérivés.

Ainsi, la composition nutritionnelle selon l'invention peut également comprendre une source de calcium, par exemple sous la forme d'un composé organique ou inorganique physiologiquement acceptable, tels que des sels de calcium inorganique (chlorure de calcium, phosphate de calcium, sulfate de calcium, oxyde de calcium, hydroxyde de calcium ou carbonate de calcium) ou des composants organiques contenant du calcium telle que la poudre de lait écrémé, le caséinate de calcium ou encore des sels organiques de calcium (citrate de calcium, maléate de calcium ou leurs mélanges).

La quantité de calcium contenue dans une composition nutritionnelle selon l'invention est adaptée pour une administration quotidienne, fournie par ladite composition, comprise entre 100 mg et 1000 mg, de préférence entre 200 mg et 700 mg et de manière tout à fait préférée entre 300 mg et 600 mg de calcium.

Une composition nutritionnelle selon l'invention peut également comprendre des vitamines, telle que la vitamine A, la vitamine D, la vitamine E, la vitamine K, la vitamine C, l'acide folique, la thiamine, la riboflavine, la vitamine B₆, la vitamine B₁₂, la niacine, la biotine ou encore l'acide pantothénique.

Une composition nutritionnelle selon l'invention peut également comprendre des éléments minéraux et des éléments trace tels que le sodium, le potassium, le phosphore, le magnésium, le cuivre, le zinc, le fer, le sélénium, le chrome et le molybdène.

Elle peut également comprendre des fibres solubles telles que l'agar-agar, un alginate, du caroube, du carragheenan , de la gomme arabique, de la gomme de guar, de la gomme de karaya, de la pectine ou de la gomme xanthane, ces fibres solubles étant sous une forme hydrolysée ou non hydrolysée.

Elle peut aussi comprendre des composés source d'énergie, notamment une ou plusieurs sources d'hydrates de carbone choisis parmi les maltodextrines, l'amidon, le lactose, le glucose, le sucrose, le fructose, le xylitol et le sorbitol.

En outre, une composition nutritionnelle selon l'invention peut également comprendre des arômes naturels ou artificiels, par exemple des arômes de fruits comme la banane, l'orange, la pêche, l'ananas ou la framboise, ou d'autres arômes végétaux comme la vanille, le cacao, le café, etc..

Comme cela a déjà été mentionné précédemment, une composition destinée à stimuler la formation de l'os ou à inhiber la résorption osseuse selon l'invention peut aussi être présentée sous la forme d'une composition pharmaceutique, comme décrit ci-dessous.

### Compositions pharmaceutiques humaines ou vétérinaires selon l'invention.

L'invention a également pour objet une composition pharmaceutique humaine ou vétérinaire pour stimuler la formation de l'os et/ou pour inhiber la résorption osseuse, caractérisée en ce qu'elle comprend, à titre de principe actif, le composé hespéridine ou l'un de ses dérivés.

En particulier, l'invention concerne l'utilisation de l'hespéridine ou de l'un de ces dérivés pour la fabrication d'une composition pharmaceutique à usage humain ou vétérinaire pour la prévention ou le traitement d'une pathologie liée à un déséquilibre du métabolisme osseux, c'est-à-dire une composition pharmaceutique apte à stimuler la formation de l'os et/ou à inhiber la résorption osseuse.

Les utilisations de l'hespéridine pour la fabrication d'une composition pharmaceutique seront décrites en relation avec les caractéristiques techniques de ladite composition pharmaceutique ci-après.

Une composition pharmaceutique selon l'invention comprend, en tant que principe actif, l'hespéridine ou l'un de ses dérivés, en une quantité adaptée pour stimuler la formation de l'os ou inhiber la résorption osseuse chez des individus nécessitant un tel traitement.

Selon un premier aspect, une composition pharmaceutique selon l'invention est utile pour stimuler la formation de l'os chez les individus jeunes, homme ou animaux, en phase de croissance, afin d'augmenter la densité osseuse atteinte au début de l'âge adulte et accroître la masse osseuse maximale (pic de masse osseuse) au début de l'âge adulte.

Selon un second aspect, une composition pharmaceutique humaine ou vétérinaire selon l'invention est utile pour prévenir la perte osseuse qui se produit avec l'âge, au cours du vieillissement.

Selon un troisième aspect, une composition pharmaceutique humaine ou vétérinaire selon l'invention est utile pour prévenir ou traiter des troubles ou des pathologies liés à un déséquilibre du rapport entre la formation de l'ose et la résorption osseuse.

Selon un quatrième aspect, une composition pharmaceutique humaine ou vétérinaire selon l'invention est utile pour traiter un déficit osseux résultant d'une fracture.

Selon un cinquième aspect, une composition pharmaceutique à destination humaine ou vétérinaire selon l'invention est utile dans le traitement de pathologies liées à un déséquilibre du remodelage osseux, telles que l'ostéoporose, la maladie de Paget, la perte osseuse ou l'ostéolyse observée à proximité d'une prothèse, les maladies de l'os métastatique, l'hypercalcémie due à un cancer, les myélomes multiples, les maladies periodontales ou encore l'ostéoarthrite.

Il peut s'agir d'une composition pharmaceutique humaine ou vétérinaire, en particulier pour le chien ou le chat, ou encore le cheval, en particulier un pur-sang.

La composition pharmaceutique selon l'invention se présente sous une forme pour l'administration orale, parentérale ou intra-veineuse.

Dans sa forme destinée à l'administration humaine, une composition pharmaceutique selon l'invention comprend avantageusement, une quantité d'hespéridine ou de l'un de ses dérivés adaptée pour une administration quotidienne du composé actif, fournie par ladite composition, comprise entre 0,01 mg et 500 mg.

Sous sa forme destinée à une administration chez l'animal, particulièrement un mammifère domestique tel que le chien ou le chat, une composition pharmaceutique selon l'invention comprend une quantité d'hespéridine ou de l'un de ses dérivés adaptée à une administration quotidienne du composé actif, fourni par ladite composition, comprise entre 1 mg et 500 mg.

Une composition pharmaceutique selon l'invention comprend l'hespéridine ou l'un de ses dérivés en association avec au moins un excipient choisi dans le groupe constitué par les excipients pharmaceutiquement acceptables.

Des techniques de préparation de compositions pharmaceutiques selon l'invention peuvent être aisément retrouvées par l'homme du métier, par exemple dans l'ouvrage Remmingston's Pharmaceutical Sciences, Mid. Publishing Co, Easton, PA, USA.

Des adjuvants, des véhicules et des excipients physiologiquement acceptables sont aussi décrits dans l'ouvrage intitulé "Handbook of Pharmaceutical Excipients, Seconde édition, American Pharmaceutical Association, 1994.

Pour formuler une composition pharmaceutique selon l'invention, l'homme du métier pourra avantageusement se référer à la dernière édition de la Pharmacopée Européenne ou de la Pharmacopée des Etats-Unis d'Amérique (USP).

L'homme du métier pourra notamment avantageusement se référer à la quatrième édition « 2002 » de la Pharmacopée Européenne, ou encore à l'édition USP 25-NF 20 de la Pharmacopée Américaine (U.S. Pharmacopeia).

Avantageusement, une composition pharmaceutique telle que définie ci-dessus est adaptée pour une administration orale, parentérale ou intraveineuse.

Lorsque la composition pharmaceutique selon l'invention comprend au moins un excipient pharmaceutique ou physiologiquement acceptable, il s'agit en particulier d'un excipient approprié pour une administration de la composition par voie orale ou d'un excipient approprié pour une administration de la composition par voie parentérale.

L'invention concerne aussi une méthode pour prévenir ou traiter un trouble lié à un déséquilibre du métabolisme osseux, en particulier un trouble associé à une perte de la masse osseuse, ladite méthode comprenant une étape au cours de laquelle on administre aux patients une quantité thérapeutiquement efficace d'hespéridine ou de l'un de ses dérivés ou encore d'une composition pharmaceutique contenant l'hespéridine ou l'un de ses dérivés.

Une composition pharmaceutique comprenant de l'hespéridine ou l'un de ses dérivés selon l'invention se présente indifféremment sous une forme solide ou sous une forme liquide.

Pour une administration orale, on préférera une composition pharmaceutique solide, sous la forme de comprimés, de capsules ou de gélules.

Sous la forme liquide, on préférera une composition pharmaceutique sous la forme d'une suspension aqueuse ou d'une suspension non aqueuse, ou encore sous la forme d'une émulsion eau-dans-huile ou huile-dans-eau.

Des formes pharmaceutiques solides peuvent comprendre, en tant que véhicules, adjuvants ou excipients, au moins un agent diluant, un arôme, un agent solubilisant, un agent lubrifiant, un agent de suspension, un agent liant, un agent désintégrant et un agent d'encapsulation.

De tels composés sont par exemple le carbonate de magnésium, le stéarate de magnésium, le talc, le lactose, la pectine, la dextrine, l'amidon, la gélatine, des matériaux cellulosiques, du beurre de cacao, etc.

Les compositions sous forme liquide peuvent comprendre également de l'eau, le cas échéant en mélange avec du propylène glycol ou du polyéthylène glycol, et éventuellement aussi des agents de coloration, des arômes, des stabilisants et des agents épaississants.

Pour la fabrication d'une composition pharmaceutique selon l'invention, l'hespéridine ou l'un de ses dérivés peut être préparée conformément à l'enseignement des différents documents de brevets cités précédemment dans la description.

De préférence, on utilisera, comme source d'hespéridine, un produit de grade pharmaceutique contenant l'hespéridine sous une forme pure, ou pratiquement pure, telle que l'hespéridine commercialisée par la Société NINGBO LIWAH PHARMACEUTICAL Co Ltd., appartenant au groupe SANJIU ENTREPRISE GROUP, qui comprend 95% en poids d'hespéridine dans l'acétate d'éthyle.

L'invention est en outre illustrée , sans pour autant être limitée, par les exemples suivants.

### Exemple 1 : Effet de l'hespéridine sur le métabolisme osseux chez la rate ovariectomisée.

L'objectif de cette expérimentation était de tester l'impact de l'hespérétine sur le métabolisme osseux de la rate ovariectomisée (OVX) par comparaison à celui de rates témoins pseudo-opérées (SH). L'hespérétine est une flavanone dont la forme glycosylée (hespéridine) est spécifique des agrumes, et plus particulièrement de l'orange. Ce flavonoïde n'est pas présent dans les autres produits végétaux. C'est sous forme d'hespéridine que les régimes expérimentaux ont été supplémentés.

### A. MATERIEL ET METHODES

Afin d'étudier l'éventuel impact de cette molécule sur le tissu osseux, 40 rates Wistar âgées de 3 mois (266 ± 2 g) ont été utilisées. Ces animaux ont été adaptés à un régime semi-synthétique de base pendant les 7 jours précédant le début de la période expérimentale. A J0, 20 rates ont été pseudo-opérées, les 20 autres ovariectomisées. Réparties en lots de 10, elles ont été soumises à divers régimes alimentaires, pendant 3 mois à l'issu desquels elles ont été sacrifiées. La dernière semaine avant l'abattage, de J85 à J90, les rates ont été mises en bilan et l'urine a été prélevée (sur une période de 24 heures). De même, la composition corporelle des rates a été mesurée. A l'abattage, le plasma et les fémurs ont été prélevés pour différentes analyses [Fémurs : résistance à la rupture, densitométrie ; Plasma : ostéocalcinémie (marqueur de l'activité ostéoblastique), taux d'hespérétine ; Urine : désoxypyridinoline (marqueur de la résorption osseuse)]. Pendant toute la durée de l'expérimentation, les rates étaient pesées une fois par semaine. La quantité journalière d'aliment distribuée à chaque animal était de 20g.

### Répartition des lots

**10 *T* SH** et **10 *T* OVX** : rates pseudo-opérées ou ovariectomisées recevant un régime témoin standard pendant 3 mois.

**10 *Hp* SH** et **10 *Hp* OVX** : rates pseudo-opérées ou ovariectomisées recevant un régime témoin standard supplémenté en hespéridine (0.5%) pendant 3 mois.

### B. RESULTATS

### B.1 Evolution pondérale et composition corporelle

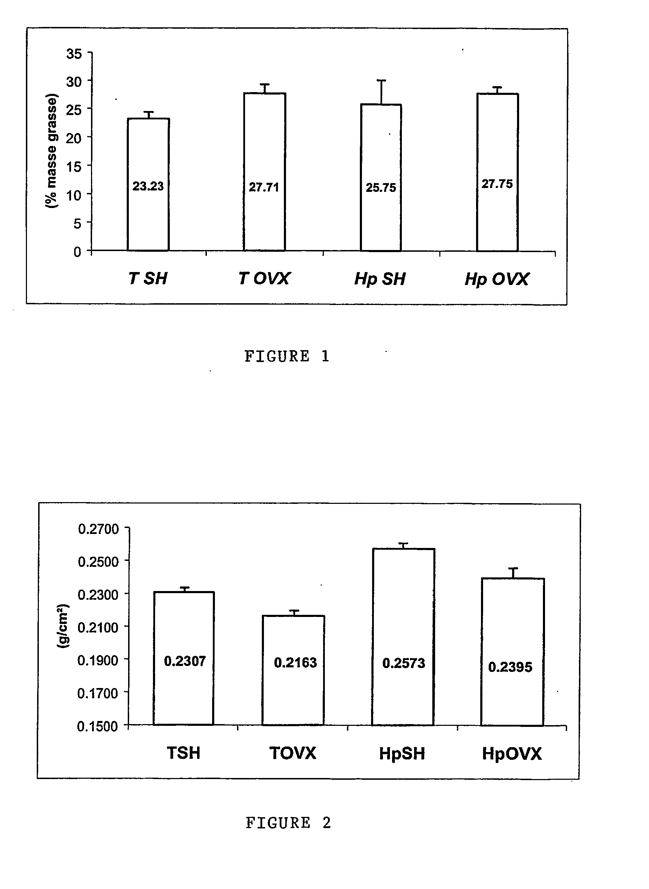

Les résultats sont présentés figure 1. Les valeurs sont exprimées en Moyennes ± SEM. Les rates ont présenté une évolution pondérale classique. Bien qu'en fin de période expérimentale le poids corporel (g) des animaux OVX était significativement supérieur par comparaison aux pseudo-opérées [*T* SH : 326.6 ± 9.4 ; *T* OVX : 363.3 ± 7 ; *Hp* SH : 335.4 ± 15.4 ; *Hp* OVX : 406.3 ± 12.7], aucune différence significative concernant la composition corporelle (% masse grasse par rapport au poids corporel total) n'a été observée entre les lots.

### B.2 Poids utérin

Le poids utérin a été évalué, afin de valider la castration. L'ovariectomie a effectivement induit une atrophie de cet organe (mg), par comparaison aux SH (p<0.01). Ce paramètre n'ayant pas été modifié par la consommation d'hespéridine, cette molécule semble donc être dépourvue d'effet utérotrophique.

**Tableau 1**

| **TSH** | **T OVX** | **Hp SH** | ***Hp OVX*** |
|---|---|---|---|
| 1031.9 ± 82.3 | 139.6 ± 10.2** | 659.4 ± 8.96 | 238.6 ± 2.23** |

### B.3 Biomécanique fémorale

Le diamètre et la longueur (mm) des fémurs n'ont pas été modifiés significativement par le traitement.

**Tableau 2 : Résistance fémorale à la rupture :**

| ***T* SH** | ***T* OVX** | ***Hp SH*** | ***Hp* OVX** |
|---|---|---|---|
| 109.5±3.3 | 102.2±2.9 | 106.3±5.3 | 112.4±3.4 |

Bien que l'ovariectomie n'ait pas significativement diminué la résistance à la rupture fémorale (N), les rates ovariectomisées et recevant un régime supplémenté en hespéridine tendent vers une amélioration de ce paramètre.

### B.3 Densitométrie

Les densités sont exprimées en g/cm².

**Tableau 3 : Densité fémorale totale (T-BMD)**

| ***T* SH** | **T OVX** | ***Hp SH*** | ***Hp* OVX** |
|---|---|---|---|
| 0.2262 ± 0.0056 | 0.2143 ± 0.0025^{ab} | 0.2501 ± 0.0028^{c} | 0.2445 ± 0.0052 |

L'ovariectomie induit une diminution significative de la densité fémorale totale des rates recevant le régime témoin, par comparaison aux pseudo-opérées. En revanche, la consommation d'hespéridine prévient totalement cette déminéralisation et, de plus, elle améliore significativement ce paramètre chez les SH.

### Densité fémorale métaphvsaire (M-BMD) (figure 2)

Les résultats relatifs au fémur entier sont également vérifiés au niveau de la zone métaphysaire essentiellement constituée d'os trabéculaire. L'amélioration de la minéralisation chez les animaux non castrés ayant consommé la molécule est également démontrée (par comparaison aux *T* SH).

### Densité fémorale diaphysaire (M-BMD) :

La zone diaphysaire est constituée d'os cortical.

**Tableau 4**

| ***T* SH** | ***T* OVX** | ***Hp* SH** | ***Hp* OVX** |
|---|---|---|---|
| 0.2019 ± 0.0044 | 0.2003 ± 0.0032 | 0.2245 ± 0.0020* | 0.2258 ± 0.0055* |

La consommation d'hespéridine améliore significativement (p<0.05) ce paramètre, par comparaison aux autres lots recevant un régime standard, aussi bien chez les rates OVX que SH.

### B.4 Biomarqueurs osseux

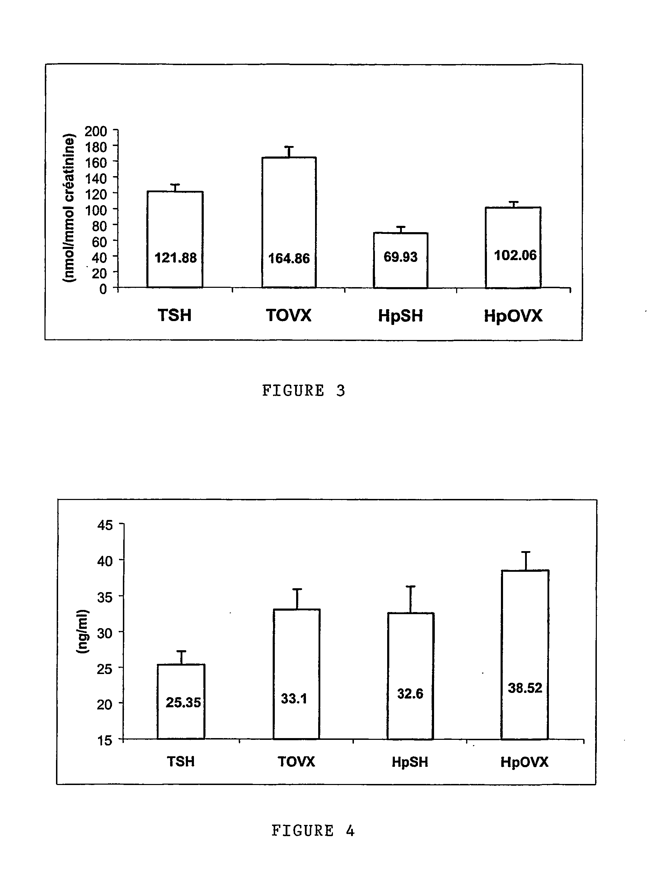

### Excrétion urinaire de désoxypyridinoline (figure 3)

L'ovariectomie induit une augmentation significative de la résorption osseuse (p<0.01 vs *T* SH). Ce phénomène est évité par la consommation d'hespéridine chez les rates OVX. De même, chez les rates *Hp* SH, l'excrétion urinaire de DPD est significativement diminuée par comparaison aux *T* SH.

### Taux plasmatiques d'ostéocalcine (figure 4)

**La consommation d'hespéridine stimule l'accrétion osseuse** (*Hp* SH *vs T* SH ; *Hp* OVX *vs T* OVX ; p<0.05), l'ostéocalcine étant un témoin de l'activité ostéoblastique.

### Taux circulants d'hespérétine

L'hespéridine est, en fait, un glycoside de l'hespérétine (hespérétine-7-Rhamnoglucoside). Le taux plasmatique moyen d'aglycone retrouvé chez les rates OVX, comme les SH, recevant les régimes supplémentés en hespéridine était de **12.53 ± 2.48 µM**. Par contre, aucune trace d'hespérétine n'a été détectée chez les rates recevant le régime témoin.

### Conclusion de l'Exemple 1

L'ensemble des résultats de cette expérimentation indique que l'hespéridine est capable de prévenir la perte osseuse consécutive à la carence oestrogénique, chez la rate ovariectomisée. Cet effet semblerait être du à une diminution de la résorption osseuse, couplée à une accélération de l'activité ostéoblastique. Un effet bénéfique de cette flavanone est même observé chez des rates intactes (pseudo-opérées) ayant donc un statut hormonal et un métabolisme osseux normal.

### Exemple 2 : Effet de l'hespérétine sur les cellules hPOB-tert (lignée immortalisée d'ostéoblastes de périoste humain, Darimont et al., 2002 .

Nous avons testé trois concentrations d'hespérétine dans le milieu de culture de hPOB ensemencées à confluence : 1, 10 et 50 µM. Ces milieux étaient par ailleurs dépourvus de facteurs de différenciation ostéoblastique (Vitamine D3 et Dexaméthasone). Le contrôle contenait du DMSO (l'hespérétine étant dissoute dans le DMSO) à 0.1 %. Les cellules ont été incubées dans ces milieux pendant 35 jours. L'activité de la phosphatase alcaline, marqueur de différenciation ostéoblastique, a été mesurée sur un lysat cellulaire en utilisant un kit commercialisé (Sigma n°247). Les cellules avaient été récoltées à 0, 4, 8 et 12 jours d'incubation. Le contenu calcique des cellules, témoin de minéralisation, a été quantifié sur lysat cellulaire après 21 jours d'incubation avec les différents milieux, à l'aide d'un kit commercialisé (Sigma n°587).

### RESULTATS

### Activité de l'ALP

L'activité de la phosphatase alcaline, donc la différenciation ostéoblastique, est stimulée par la présence d'hespéritine dans le milieu, de façon dose-dépendante. Les doses 10 et 50 µM augmentent ce processus dés le 4ème jour d'expérimentation, un palier étant atteint au 8ème jour d'incubation. Si l'hespérétine à 50 µM s'avère la plus efficace, une concentration de 1 µM, au contraire, serait dépourvue d'effet, par comparaison aux cellules non traitées.

### Accumulation du calcium dans les cellules:

Après 21 jours d'incubation, seule la dose à 50µM d'hespérétine semble stimuler le dépôt calcique, par comparaison au contenu de calcium dans les cellules contrôles (0.1 % DMSO).

### Exemple 3 : Effet de l'hespéridine sur le métabolisme osseux chez la rate ovariectomisée âgée de six mois.

### A. Matériel et Méthodes

L'expérimentation a été réalisée sur 48 rates Wistar âgées de 6 mois : 24 ovariectomisées (OVX) et 24 pseudo-opérées (SH).

Les animaux ont été logés dans des cages individuelles à une température contrôlée de 21 ± 1 °C, selon un cycle nyctéméral de 12h-12h.

Après l'intervention chirurgicale à J0, les animaux ont été accoutumés pendant sept jours. A l'issue de cette période d'adaptation, les rongeurs ont été répartis sur la base des paramètres pondéraux en 4 lots homogènes et ont reçu quotidiennement pendant 72 jours 22 g des régimes suivants :
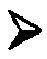 lot SH : 12 rattes pseudo-opérées alimentées avec le régime contrôle
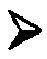 lot OVX : 12 rattes ovariectomisées ayant reçu le régime contrôle
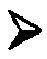 lot SH Hp : 12 rattes pseudo-opérées alimentées avec le régime hespéridine
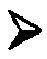 lot OVX Hp : 12 rattes ovariectomisées ayant reçu le régime hespéridine.

Au sacrifice (J90), les animaux ont été anesthésiés par injection intra-péritonéale de chloral (Fluka ; solution à 8% ; 0,4 ml/100g de poids vif). Des prélèvements sanguins ont été pratiqués au niveau de l'aorte abdominale. L'utérus a été pesé.

Les fémurs ont été prélevés et débarrassés des tissus mous adjacents, puis conservés dans de l'éthanol à 80% pour la détermination de la densité osseuse.

### B. RESULTATS

### 1. Consommation alimentaire

La quantité d'aliments distribuée a été calculée sur la base du niveau de la consommation moyenne la plus faible la semaine précédant l'essai (lot SH).

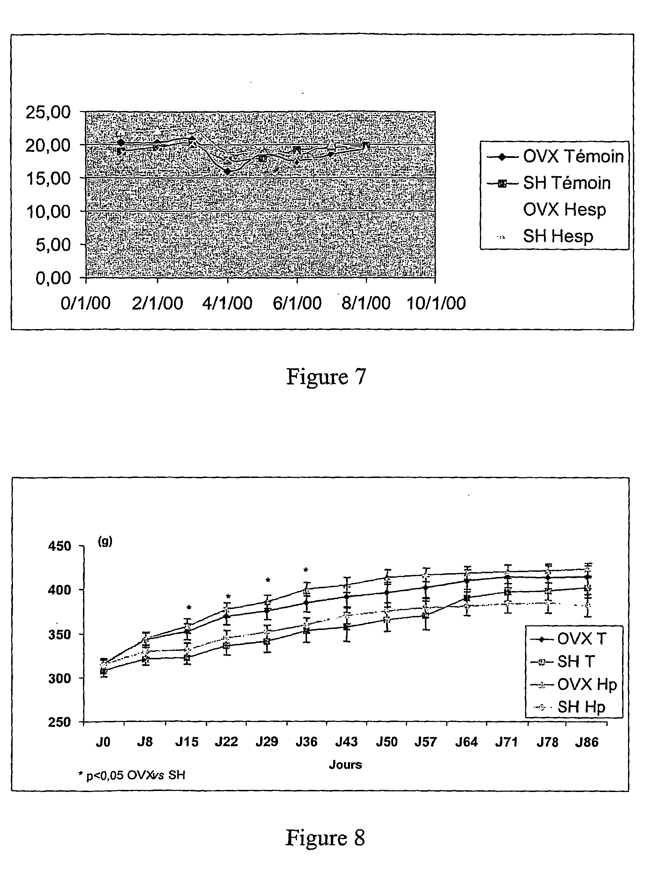

Les résultats sont représentés sur la Figure 7.

Les résultats de la Figure 7 montrent qu' aucune différence significative au niveau de l'alimentation des animaux des différents lots n'est observée.

### 2. Evolution pondérale

Les résultats de l'évolution pondérale sont représentés sur la Figure 8.

Le profil pondéral des animaux de tous les lots présente une évolution similaire, à savoir une augmentation entre le début et la fin de l'expérience, quel que soit le groupe considéré.

### 3. Composition corporelle

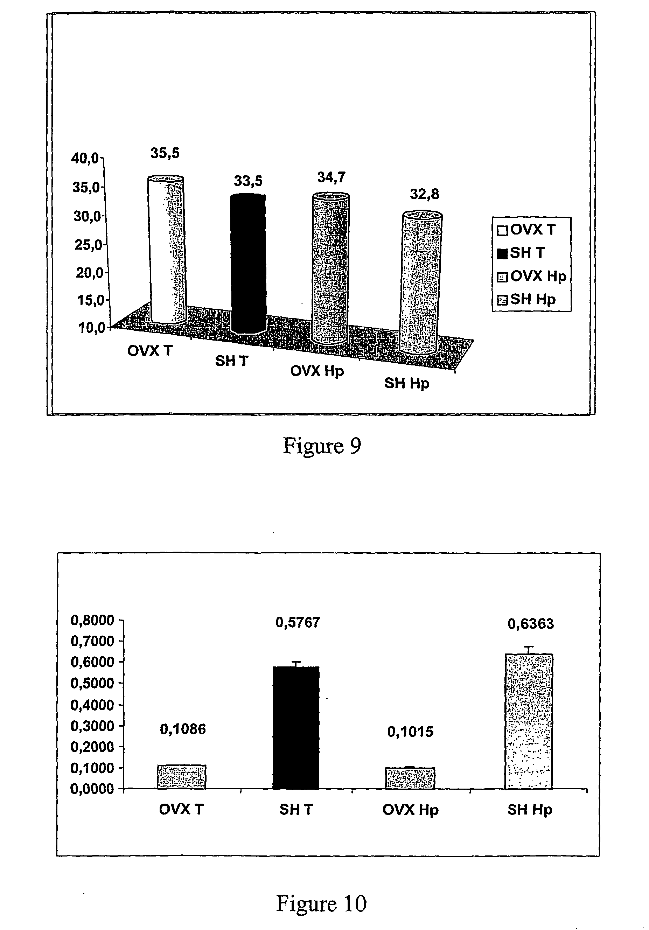

Les résultats de mesure de la composition corporelle sont représentés sur la Figure 9.

La composition corporelle des animaux dans les différents groupes n'a pas été modifiée de façon significative, quel que soit le traitement subi par les individus.

### 4. Poids utérin

Les résultats sont représentés sur la Figure 10.

L'atrophie utérine consécutive à l'ablation ovarienne n'est pas évitée par la consommation d'hespéridine.

Les résultats présentés à la Figure 10 montrent donc que l'hespéridine est dépourvue d'effet utérotrophique.

### 5. Densité minérale osseuse

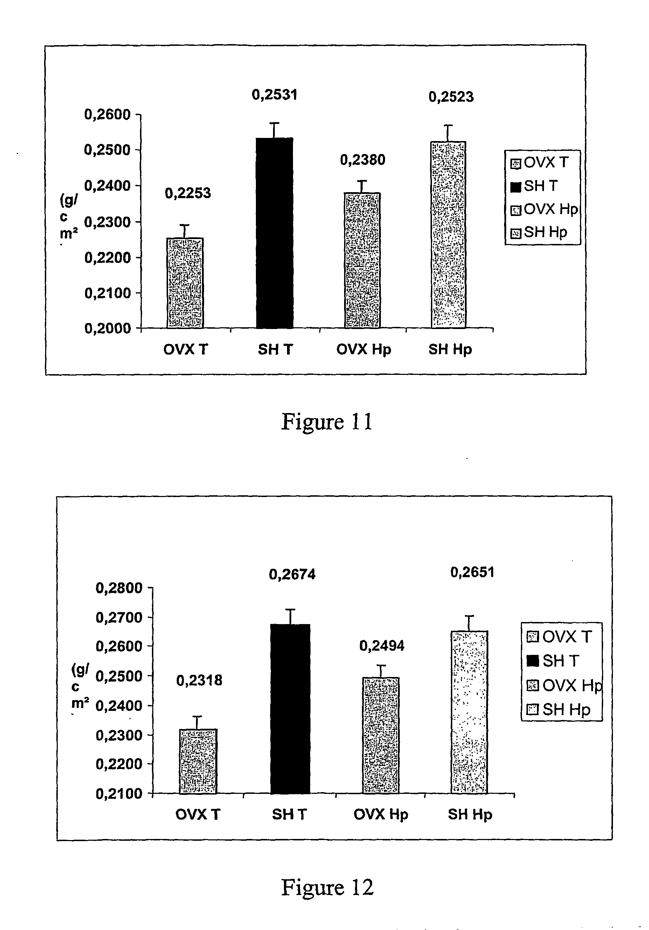

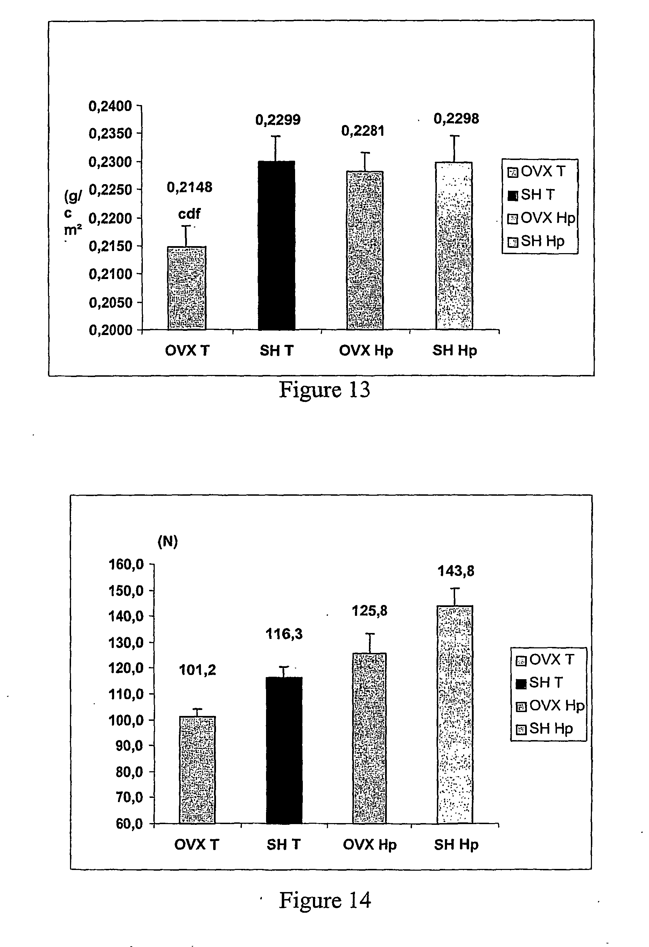

Les résultats de mesure de densité minérale osseuse sont représentés sur les figures 11, 12 et 13. Les résultats de mesure de la densité minérale fémorale totale sont représentés sur la Figure 11. Les résultats de mesure de la densité minérale de la métaphyse proximale fémorale sont représentés sur la Figure 12. Les résultats de mesure de la densité minérale diaphysaire fémorale sont représentés sur la Figure 13.

La castration s'est traduite par une déminéralisation du fémur, mise en évidence par une densité minérale osseuse totale (g/cm²) réduite. Ce processus est évité (au moins partiellement) par la consommation d'hespéridine.

Un profil similaire est démontré au niveau trabéculaire (Fig. 12), ainsi que dans l'os cortical (Fig. 13).

### 6. Propriétés biomécaniques

On a analysé les propriétés mécaniques du fémur des individus des différents lots d'animaux. Les résultats sont représentés sur la Figure 14.

Les propriétés biomécaniques du fémur sont détériorées par la carence oestrogénique. La consommation d'hespéridine (OVX Hp) a permis de les préserver, voire de les améliorer chez les animaux intacts.

En conclusion, l'ensemble des résultats de l'exemple 3 montrent que l'hespéridine possède une activité ostéoprotectrices, dans un modèle expérimental de la perte osseuse liée au vieillissement.

## Revendications

1. Utilisation du composé hespéridine ou de l'un de ses dérivés choisis parmi l'hespérétine, une méthyl-hespéridine, un conjugué d'hespérétine et de sulfate ou de glucuronide et l'α-glucosyl-hespéridine comportant une chaîne de 1 à 20 résidus de glucose liés entre eux par une liaison 1,4, la chaîne de résidus glucose étant liée elle-même par une liaison de type 1,4 en position 4 du résidu glucose de l'hespéridine, en tant que composé actif sur la stimulation de la formation de l'os et/ou sur l'inhibition de la résorption osseuse, pour la fabrication d'une composition destinée à stimuler la formation de l'os et/du à inhiber la résorption osseuse chez l'homme ou l'animal.

2. Utilisation selon la revendication 1, **caractérisée en ce que** ladite composition est une composition nutritionnelle adaptée pour une administration orale.

3. Utilisation selon la revendication 2, **caractérisée en ce que** ladite composition nutritionnelle est destinée à stimuler la formation de l'os chez les individus jeunes en phase de croissance.

4. Utilisation selon la revendication 2, **caractérisée en ce que** ladite composition nutritionnelle est destinée à prévenir la perte osseuse qui se produit avec le vieillissement

5. Utilisation selon la revendication 2, **caractérisée en ce que** ladite composition nutritionnelle est destinée à prévenir ou traiter des troubles liés à un déséquilibre du rapport entre la formation de l'os et la résorption osseuse.

6. Utilisation selon la revendication 2, **caractérisée en ce que** ladite composition nutritionnelle est destinée à traiter un déficit osseux résultant d'une fracture.

7. Utilisation selon la revendication 6., **caractérisée** ne ce que ladite composition nutritionnelle est destinée à prévenir ou à traiter des pathologies choisies parmi l'ostéoporose, la maladie de Paget, la perte osseuse ou l'ostéolyse observée à proximité d'une prothèse, les maladies de l'os métastatique, l'hypercalcémie due à un cancer, les myélomes multiples, les maladies periodontales ou l'ostéoarthrite.

8. Utilisation selon l'une des revendications 2 à 7, **caractérisée en ce** ladite composition nutritionnelle se présente sous forme de boissons, y compris des jus, préférentiellement des jus de fruits, des yaourts, des glaces, des fromages, des produits cuits tels que le pain, les biscuits et les gâteaux, des produits laitiers, des desserts, des produits de confiserie, des barres de céréales, des céréales pour le petit déjeuner, des produits d'assaisonnement des aliments, des salades de fruits ou des compotes de fruits.

9. Utilisation selon l'un des revendications 2 à 7, **caractérisée en ce que** ladite composition nutritionnelle se présente sous la forme d'un produit destiné à l'alimentation animale, sous forme humide, semi-humide ou sèche.

10. Utilisation selon l'une des revendications 2 à 9, **caractérisée en ce que** le composé hispéridine ou l'un de ses dérivés se présente sous la forme d'un produit d'extraction obtenu à partir de la peau ou de la pulpe d'un agrume.

11. Utilisation selon l'une des revendications 2 à 10, **caractérisée en ce que** ladite composition nutritionnelle est adaptée à une administration orale d'une quantité quotidienne comprise entre 0,01 à 500 mg du composé hispéridine ou de l'un de ses dérivés.

12. Utilisation selon la revendication 1, **caractérisée en ce** ladite composition est une composition pharmaceutique humaine ou vétérinaire.

13. Utilisation selon la revendication 12, **caractérisée en ce que** ladite composition pharmaceutique est destinée à stimuler la formation de l'os chez l'individu jeune en phase de croissance.

14. Utilisation selon la revendication 12, **caractérisée en ce que** ladite composition pharmaceutique est destinée à prévenir la perte osseuse qui se produit au cours du vieillissement

15. Utilisation selon la revendication 12, **caractérisée en ce que** ladite composition pharmaceutique est destinée à prévenir ou traiter une pathologie liée à un déséquilibre du rapport entre la formation de l'ose et la résorption osseuse.

16. Utilisation selon la revendication 12, **caractérisée en ce que** ladite composition pharmaceutique est destinée à traiter un déficit osseux résultant d'une fracture.

17. Utilisation selon la revendication 12, **caractérisée en ce que** ladite composition est destinée à prévenir ou traiter une pathologie choisie parmi que l'ostéoporose, la maladie de Paget, la perte osseuse ou l'ostéolyse observée à proximité d'une prothèse, les maladies de l'os métastatique, l'hypercalcémie due à un cancer, les myélomes multiples, les maladies periodontales ou l'ostéoarthrite.

18. Utilisation selon l'une des revendications 12 à 17, **caractérisée en ce que** ladite composition pharmaceutique se présente sous une forme pour l'administration orale, parentérale ou intra-veineuse.

19. Utilisation selon l'une des revendications 12 à 17, **caractérisée en ce que** ladite composition pharmaceutique est adaptée à une administration orale d'une quantité quotidienne comprise entre 0,01 à 500 mg du composé hispéridine ou de l'un de ses dérivés.

## Claims

1. The use of the compound hesperidin or of one of its derivatives selected from hesperetin, methyl-hesperidin, a conjugate of hesperetin and sulfate or glucuronide and α-glucosyl-hisperidin having a chain of 1 to 20 glucose residues linked together by a 1,4 bond, the chain of glucose residues being linked itself by a bond of the 1,4 type in 4 position of the glucose residue of hesperidin, as an active compound on the stimulation of bone formation and/or the inhibition of bone resorption for the manufacture of a composition designed to stimulate bone formation and/or inhibit bone resorption in man or animals.

2. The use according to Claim 1, **characterised in that** said composition is a nutritional composition suitable for oral administration.

3. The use according to Claim 2, **characterised in that** said nutritional composition is designed to stimulate bone formation in young individuals in the growth phase.

4. The use according to Claim 2, **characterised in that** said nutritional composition is designed to prevent the bone loss which occurs with ageing.

5. The use according to Claim 2, **characterised in that** said nutritional composition is designed to prevent or treat disorders linked to an imbalance in the relationship between bone formation and bone resorption.

6. The use according to Claim 2, **characterised in that** said nutritional composition is designed to treat a bone deficit resulting from a fracture.

7. the use according to Claim 6, **characterised in that** said nutritional composition is designed to prevent or treat diseases selected from osteoporosis, Paget's disease, bone loss or osteolysis observed close to a prosthesis, metastatic bone diseases, hypercalcemia due to a cancer, multiple myelomas, periodontal diseases or osteoarthritis.

8. The use according to one of the Claims 2 to 7, **characterised in that** said nutritional composition is available in the form of drinks, including juices, preferably fruit juices, yoghurts, ice creams, cheeses, baked products such as bread, biscuits and cakes, dairy products, desserts, confectionery products, cereal bars, breakfast cereals, food seasoning products, fruit salads or stewed fruit.

9. The use according to one of the Claims 2 to 7, **characterised in that** said nutritional composition is available in the form of a product designed for animal feed, in a wet, semi-wet or dry form.

10. The use according to one of the Claims 2 to 9, **characterised in that** the compound hesperidin or one of its derivatives is available in the form of an extraction product obtained from the peel or the pulp of a citrus fruit.

11. The use according to one of the Claims 2 to 10, **characterised in that** said nutritional composition is adapted for oral administration of a daily quantity included between 0.01 and 500 mg of the compound hesperidin or of one of its derivatives.

12. The use according to Claim 1, **characterised in that** said composition is a human pharmaceutical or veterinary composition.

13. The use according to Claim 12, **characterised in that** said pharmaceutical composition is designed to stimulate bone formation in the young individual in the growth phase.

14. The use according to Claim 12, **characterised in that** said pharmaceutical composition is designed to prevent the bone loss which occurs in the course of ageing.

15. The use according to Claim 12, **characterised in that** said pharmaceutical composition is designed to prevent or treat a disease linked to an imbalance in the relationship between bone formation and bone resorption.

16. The use according to Claim 12, **characterised in that** said pharmaceutical composition is designed to treat a bone deficit resulting from a fracture.

17. The use according to Claim 12, **characterised in that** said composition is designed to prevent or treat a disease selected from osteoporosis, Paget's disease, bone loss or the osteolysis observed close to a prosthesis, metastatic bone diseases, the hypercalcemia due to a cancer, multiple myelomas, periodontal diseases or osteoarthritis.

18. The use according to one of the Claims 12 to 17, **characterised in that** said pharmaceutical composition is available in a form for oral, parenteral or intravenous administration.

19. The use according to one of the Claims 12 to 17, **characterised in that** the pharmaceutical composition is suitable for an oral administration of a daily quantity included between 0.01 and 500 mg of the compound hesperidin or of one of its derivatives.

## Patentansprüche

1. Verwendung der Verbindung Hesperidin oder eines seiner Derivate, die aus Hesperetin, einem Methylhesperidin, einem Konjugat von Hesperetin und Sulfat oder Glucuronid von α-Glucosylhesperidin, das eine Kette von 1 bis 20 Glucoseresten umfasst, die durch eine 1,4-Bindung miteinander verknüpft sind, wobei die Kette der Glucosereste wiederum durch eine Bindung des 1,4-Typs in Position 4 des Glucoserestes von Hesperidin gebunden ist, als Wirkstoff für die Stimulierung der Knochenbildung und/oder für die Hemmung der Knochenresorption für die Herstellung einer Zusammensetzung zum Stimulieren der Knochenbildung und/oder zum Hemmen der Knochenresorption bei Mensch oder Tier.

2. Verwendung gemäß Anspruch 1, **dadurch gekennzeichnet, dass** die Zusammensetzung eine Nährzusammensetzung ist, die für die orale Verabreichung geeignet ist.

3. Verwendung gemäß Anspruch 2, **dadurch gekennzeichnet, dass** die Nährzusammensetzung dazu dient, die Knochenbildung bei jungen Individuen in der Wachstumsphase zu stimulieren.

4. Verwendung gemäß Anspruch 2, **dadurch gekennzeichnet, dass** die Nährzusammensetzung dazu dient, den Knochenverlust, der mit der Alterung einhergeht, zu verhindern.

5. Verwendung gemäß Anspruch 2, **dadurch gekennzeichnet, dass** die Nährzusammensetzung dazu dient, Störungen in Verbindung mit einem Ungleichgewicht des Verhältnisses zwischen der Knochenbildung und der Knochenresorption zu verhindern oder zu behandeln.

6. Verwendung gemäß Anspruch 2, **dadurch gekennzeichnet, dass** die Nährzusammensetzung dazu dient, ein Knochendefizit, der sich aus einer Fraktur ergibt, zu behandeln.

7. Verwendung gemäß Anspruch 6, **dadurch gekennzeichnet, dass** die Nährzusammensetzung dazu dient, Pathologien zu verhindern oder zu behandeln, die aus Osteoporose, Morbus Paget, Knochenverlust oder Osteolyse, der bzw. die in der Nähe einer Prothese beobachtet wird, Knochenmetastasen, Hypercalcämie aufgrund von Krebs, multiplen Myelomen, periodontalen Krankheiten oder Osteoarthritis ausgewählt sind.

8. Verwendung gemäß einem der Ansprüche 2 bis 7, **dadurch gekennzeichnet, dass** die Nährzusammensetzung in Form von Getränken einschließlich Säften, vorzugsweise Fruchtsäften, Joghurts, Speiseeis, Käse, Backwaren, wie Brot, Keksen und Kuchen, Milchprodukten, Desserts, Süßwaren, Getreideriegeln, Frühstücksriegeln, Produkten zum Würzen von Nahrungsmitteln, Obstsalaten oder Fruchtmusen vorliegt.

9. Verwendung gemäß einem der Ansprüche 2 bis 7, **dadurch gekennzeichnet, dass** die Nährzusammensetzung in Form eines Produkts zur Tierernährung in feuchter, halbfeuchter oder trockener Form vorliegt.

10. Verwendung gemäß einem der Ansprüche 2 bis 9, **dadurch gekennzeichnet, dass** die Verbindung Hesperidin oder eines seiner Derivate in Form eines Extraktionsprodukts vorliegt, das aus der Schale oder dem Fleisch einer Zitrusfrucht erhalten wurde.

11. Verwendung gemäß einem der Ansprüche 2 bis 10, **dadurch gekennzeichnet, dass** die Nährzusammensetzung für eine orale Verabreichung einer täglichen Menge zwischen 0,01 und 500 mg der Verbindung Hesperidin oder eines seiner Derivate geeignet ist.

12. Verwendung gemäß Anspruch 1, **dadurch gekennzeichnet, dass** die Zusammensetzung eine human- oder veterinärmedizinische pharmazeutische Zusammensetzung ist.

13. Verwendung gemäß Anspruch 12, **dadurch gekennzeichnet, dass** die pharmazeutische Zusammensetzung dazu dient, die Knochenbildung bei jungen Individuen in der Wachstumsphase zu stimulieren.

14. Verwendung gemäß Anspruch 12, **dadurch gekennzeichnet, dass** die pharmazeutische Zusammensetzung dazu dient, den Knochenverlust, der mit der Alterung einhergeht, zu verhindern.

15. Verwendung gemäß Anspruch 12, **dadurch gekennzeichnet, dass** die pharmazeutische Zusammensetzung dazu dient, Störungen in Verbindung mit einem Ungleichgewicht des Verhältnisses zwischen der Knochenbildung und der Knochenresorption zu verhindern oder zu behandeln.

16. Verwendung gemäß Anspruch 12, **dadurch gekennzeichnet, dass** die pharmazeutische Zusammensetzung dazu dient, ein Knochendefizit, das sich aus einer Fraktur ergibt, zu behandeln.

17. Verwendung gemäß Anspruch 12, **dadurch gekennzeichnet, dass** die Zusammensetzung dazu dient, eine Pathologie zu verhindern oder zu behandeln, die aus Osteoporose, Morbus Paget, Knochenverlust oder Osteolyse, der bzw. die in der Nähe einer Prothese beobachtet wird, Knochenmetastasen, Hypercalcämie aufgrund von Krebs, multiplen Myelomen, periodontalen Krankheiten oder Osteoarthritis ausgewählt sind.

18. Verwendung gemäß einem der Ansprüche 12 bis 17, **dadurch gekennzeichnet, dass** die pharmazeutische Zusammensetzung in einer Form für die orale, parenterale oder intravenöse Verabreichung vorliegt.

19. Verwendung gemäß einem der Ansprüche 12 bis 17, **dadurch gekennzeichnet, dass** die pharmazeutische Zusammensetzung für eine orale Verabreichung einer täglichen Menge zwischen 0,01 und 500 mg der Verbindung Hesperidin oder eines seiner Derivate geeignet ist.
